(19) Europäisches Patentamt
European Patent Office
Office européen des brevets

(11) Publication number : 0 212 868 B1

(12) **EUROPEAN PATENT SPECIFICATION**

(45) Date of publication of patent specification : 13.12.89

(51) Int. Cl.[4] : C 07 C 97/10, C 07 D317/66, C 07 C103/38

(21) Application number : 86305769.1

(22) Date of filing : 28.07.86

(54) **Process for preparing 2-acyl-3,4-dialkoxyanilines.**

(30) Priority : 29.07.85 US 759973

(43) Date of publication of application : 04.03.87 Bulletin 87/10

(45) Publication of the grant of the patent : 13.12.89 Bulletin 89/50

(84) Designated contracting states : AT BE CH DE FR GB IT LI LU NL SE

(56) References cited :
EP-A- 0 138 492
CH-A- 194 648
CH-A- 466 272
US-A- 3 235 456

(73) Proprietor : ORTHO PHARMACEUTICAL CORPORATION
U.S. Route 202 P.O. Box 300
Raritan New Jersey 08869-0602 (US)

(72) Inventor : Conley, Richard A.
RD No. 1 Box 245
Annandale New Jersey 08801 (US)
Inventor : Barton, Donald L.
Rd No. 1 Box 143
Frenchtown New Jersey 08825 (US)

(74) Representative : Jones, Alan John et al
CARPMAELS & RANSFORD 43 Bloomsbury Square
London, WC1A 2RA (GB)

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue St-Denis, 75001 Paris, France

## Description

The present invention relates to a method of preparing 2-acyl-3,4-dialkoxyanilines.

The 2-acyl-3,4-dialkoxyanilines which are the subject of this invention have the following formula :

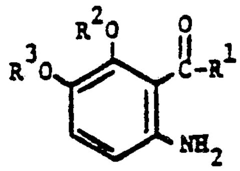

wherein $R_1$, $R_2$ and $R_3$ are lower alkyl having 1-5 carbon atoms and $R_2$ and $R_3$ when taken together are methylenedioxy.

The preparation of the 2-acyl-3,4-dialkoxyanilines is illustrated by the following schematic diagram :

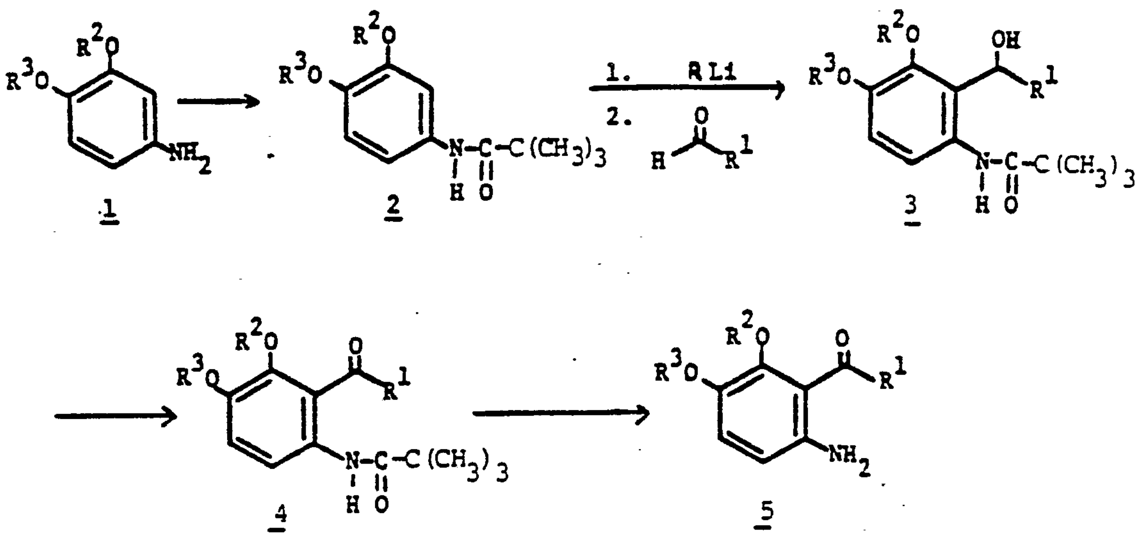


1 2 3 4 5

wherein $R_1$, $R_2$ and $R_3$ are lower alkyl having 1-5 carbon atoms and $R_2$ and $R_3$ when taken together are methylenedioxy and R is n-butyl, s-butyl or t-butyl.

In each of the steps in the process, the products are isolated where indicated and characterized by techniques known to those skilled in the art.

As can be seen from the above diagram, the first step in the synthesis of the 2-acyl-3,4-dialkoxyanilines involves the reaction of a 3,4-dialkoxyaniline (1) with a pivalic acid halide or anhydride such as pivaloyl chloride, pivaloyl bromide, pivaloyl fluoride, or pivalic anhydride to form a 3',4'-dialkoxy-2,2-dialkylpropioanilide (2). The reaction is carried out in a suitable solvent, such as, for example, methylene chloride or tetrahydrofuran, in the presence of a suitable base such as sodium hydroxide, potassium hydroxide or triethylamine. The reaction may be carried out at temperatures between 5 °C and room temperature. The anilide (2) is then reacted with an organolithium reagent such as, for example, n-butyllithium, s-butyllithium or t-butyllithium, to form in situ the corresponding dilithio intermediate which is then reacted with an aliphatic aldehyde to form the corresponding alcohol (3). The reaction is carried out in a suitable solvent such as tetrahydrofuran or diethyl ether. The reaction with the organolithium compound is carried out at a temperature between about 0 °C and 10 °C. The aldehyde is added to the reaction mixture at a temperature between — 60 °C and — 78 °C and the mixture is allowed to warm to room temperature. The alcohol (3) is then oxidized with an oxidizing agent such as, for example, chromic acid or pyridinium chlorochromate to give the 2'-acyl-3',4'-dialkoxy-2,2-dialkylpropioanilide (4). The oxidation reaction is carried out in a suitable solvent such as, for example, methylene chloride or chloroform, at a temperature between about 0 °C and 50 °C. The anilide (4) is then hydrolyzed with acid to form the 2-acyl-3,4-dialkoxyaniline (5). Suitable acids which can be employed include sulfuric acid and hydrochloric acid.

Where not otherwise indicated herein, lower alkyl shall mean an alkyl group containing 1-4 carbon atoms.

The 2-acyl-3,4-dialkoxyanilines are useful as intermediates in the preparation of the 5,6-dialkoxy-4-alkyl-2(1H)-quinazolinones which are the subject of U.S. Patent No. 4 490 374, the pertinent subject matter of which is incorporated herein by reference. The 5,6-dialkoxy-4-alkyl-2(1H)-quinazolinones are useful as cardiotonic agents.

The process of this invention eliminates the regioisomer problems associated with the preparation of the quinazolinediones disclosed in U.S. Patent No. 4 490 374 while shortening the overall synthesis. Some of the intermediates prepared in the synthesis of the 2-acyl-3,4-dialkoxyanilines are novel compounds and as such are part of the present invention.

All of the starting materials employed in the process are either known materials or can be readily made from known materials by one skilled in the art.

The following examples describe the invention in greater particularity and are intended to be a way of illustrating but not limiting the invention.

## Example 1

3′,4′-Dimethoxy-2,2-dimethylpropioanilide (2)

A solution of 368 g (2.4 mol) of 3,4-dimethoxyaniline in 1 400 mL of methylene chloride was prepared and treated with Darco at 25 °C for 20 minutes. After filtering through a Hyflo bed, 1 320 mL of 2N sodium hydroxide was added and the reaction mixture was cooled to 10 °C. Pivaloyl chloride (2.4 mol-296 mL) was added over 1 hour and the reaction mixture was then stirred for an additional hour at room temperature. The methylene chloride layer was separated, dried with magnesium sulfate, and Darco treated. The resultant methylene chloride solution was heated to reflux (45 °C) and 1 500 mL of heptane was added. The reaction was then cooled to 30 °C and 1 000 mL of 10 : 90 methylene chloride/heptane was added with cooling to 0 °C. Filtration gave 467 g (82 %) of the desired product, mp 126-128 °C. NMR ($CDCl_3$) δ 1.30 (s, 9H, C—$C\underline{H}_3$), 3.83 (s, 3H, $OC\underline{H}_3$), 3.87 (s, 3H, $OC\underline{H}_3$), 6.80 (m, 2H, Ar$\underline{H}$), 7.27 (br s, 1H, N$\underline{H}$), 7.42 (m, 1H, Ar$\underline{H}$).

## Example 2

2′-(1-Hydroxyethyl)-3′,4′-dimethoxy-2,2-dimethyl-propioanilide (3)

A solution of 10.0 g (42.2 mmol) of 3′,4′-dimethoxy-2,2-dimethylpropioanilide (2) in 130 mL of dry tetrahydrofuran was prepared under a nitrogen atmosphere and cooled to 0 °C. n-Butyllithium (52.0 mL-84.4 mmol of 1.62 M) was added keeping the temperature below 10 °C during the addition. The reaction was stirred for 1 hr at 0-5 °C and then cooled to —78 °C. A solution of 6.7 mL (84.4 mmol) of acetaldehyde in 20 mL of tetrahydrofuran was added while keeping the temperature below —60 °C. The resulting solution was allowed to warm to room temperature and was diluted with 50 mL of saturated sodium chloride solution. The layers were separated and the aqueous layer was reextracted with methylene chloride (2 × 25 mL). The combined organic layers were dried over magnesium sulfate, Darco treated, and evaporated to an oil which was crystallized from 30 mL of ethyl acetate-hexane (15 : 85). Filtration and drying under vacuum at 60-70 °C gave 8.6 g (73 %) of alcohol (3), mp 112-114 °C. NMR ($CDCl_3$) δ 1.28 (s, 9H, C—$C\underline{H}_3$), 1.50 (d, 3H, $\overset{OH}{\overset{|}{C}}H—C\underline{H}_3$), 3.15 (d, 1H, O$\underline{H}$-), 3.77 (s, 3H, $OC\underline{H}_3$), 3.80 (s, 3H, $OC\underline{H}_3$), 5.57 (m, 1H, —$\overset{OH}{\overset{|}{C}}\underline{H}—CH_3$), 6.73 (d, 1H, J = 10 Hz, Ar$\underline{H}$), 7.90 (d, 1H, J = 10 Hz, Ar$\underline{H}$), 9.60 (br s, 1H, N$\underline{H}$).

## Example 3

2′-Acetyl-3′-4′-dimethoxy-2,2-dimethylpropioanilide (4)

A solution of 80.0 g (284 mmol) of 2′-(1-hydroxyethyl)-3′-4′-dimethoxy-2,2-dimethylpropioanilide (3) in 200 mL of methylene chloride was prepared and cooled to 10 °C. Chromic acid reagent (322 mL — prepared by slowly adding 136 g of concentrated sulfuric acid to 100 g of sodium dichromate in 100 mL of water and diluting to 500 mL) was added and the reaction was refluxed at 40 °C for 1 hour. The reaction was cooled, an additional 322 mL of chromic acid reagent was added, and the reaction was refluxed for another hour. The reaction was cooled to room temperature and Darco treated. The Darco residue was washed with 100 mL of water and 500 mL of methylene chloride. The reaction mixture was extracted with 3 × 1 L of methylene chloride, dried over magnesium sulfate, and Darco treated. Evaporation gave 60.5 g (77 %) of the product.

NMR ($CDCl_3$) δ 1.28 (s, 9H, C—$C\underline{H}_3$), 2.62 (s, 3H, $\overset{O}{\overset{\|}{C}}—C\underline{H}_3$), 3.88 (s, 6H, $OC\underline{H}_3$), 7.02 (d, 1H, J = 9 Hz, Ar$\underline{H}$) 8.07 (d. 1H, J = 9 Hz, Ar$\underline{H}$), 9.88 (s, 1H, N$\underline{H}$).

## Example 4

2-Acetyl-3,4-dimethoxyaniline (5)

A solution of 4.0 g (14 mmol) of 2'-acetyl-3',4'-dimethoxy-2,2-dimethylpropioanilide (4) in 25 ml of 25 % sulfuric acid and 25 ml of methanol was prepared and refluxed for 20 hours. The reaction was cooled to room temperature, diluted with 100 mL of water and then extracted with 100 mL of methylene chloride. The aqueous layer was adjusted to pH 9 and extracted with 100 mL of methylene chloride. This methylene chloride solution was dried over sodium sulfate and decolorized with charcoal. Evaporation gave 2.4 g (85 %) of the product as a brown solid. NMR ($CDCl_3$) δ 2.60 (s, 3H, C—C$\underline{H}_3$), 3.80 (s, 3H, OC$\underline{H}_3$), 3.87 (s, 3H, OC$\underline{H}_3$), 5.00 (s, 2H, N$\underline{H}_2$), 6.33 (d, 1H, J = 8 Hz, Ar$\underline{H}$), 6.90 (d, 1H, J = 8 Hz, Ar$\underline{H}$).

Preparation of 5,6-dimethoxy-4-methyl-2(1H)-quinazolinone from a 2-acyl-3,4-dialkoxyaniline.

5,6-Dimethoxy-4-methyl-2(1H)-quinazolinone Hydrochloride Hydrate

2-Acetyl-3,4-dimethoxyaniline (15.00 g, 0.077 mole) in acetic acid (375 mL) was treated with potassium isocyanate (15 g) portionwise over 1-3 hours. The mixture was stirred unter nitrogen atmosphere at 25-35 °C for 16 hours. The precipitate was collected by filtration, washed with water (100 mL) and acetone (100 mL) and air dried to give a solid (12.6 g, 74 %) ? The solid was suspended in water (175 mL), warmed to 70 °C and concentrated hydrochloric acid (175 mL) was added. The temperature was raised to 110 °C until complete solution occurred. Hot filtration, subsequent cooling to 15 °C with stirring and filtration of the resulting precipitate provided the title compound as yellow crystals. Washing with 6N hydrochloric acid (30 mL) and acetone (120 mL) and drying gave 12.68 g (71 %) of the hydrate of 5,6-dimethoxy-4-methyl-2(1H)-quinazolinone hydrochloride, mp 203-205 °C.

## Claims

1. A method for preparing a compound of the formula

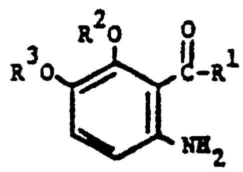

which comprises reacting a substituted aniline of the formula

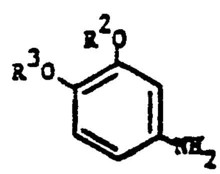

with a pivalic acid compound of the formula

$$(CH_3)_3C-\overset{O}{\overset{\|}{C}}-X$$

to form an anilide of the formula

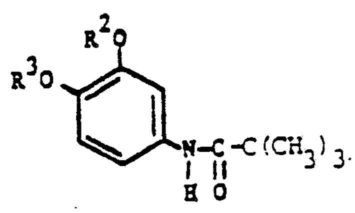

reacting the anilide first with an organolithium compound of the formula

R—Li

and reacting the product formed with an aldehyde of the formula

$R_1$—CHO

to form an anilide of the formula

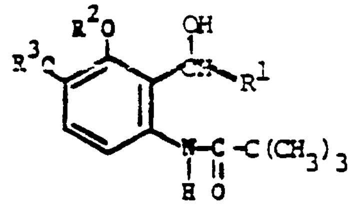

reacting the anilide with an oxidizing agent to form an anilide of the formula

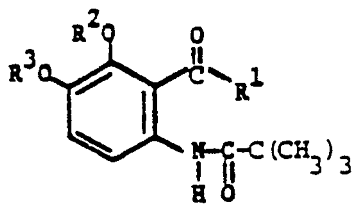

and hydrolyzing the anilide with acid, wherein $R_1$, $R_2$ and $R_3$ are lower alkyl, $R_2$ and $R_3$ when taken together are methylenedioxy and R is n-butyl, s-butyl or t-butyl and X is chloro, bromo, fluoro or $OCOC(CH_3)_3$.

2. The process of claim 1 wherein the acid halide is pivaloyl chloride.
3. The process of claim 1 wherein the organolithium compound is n-butyllithium.
4. The process of claim 1 wherein the aldehyde is acetaldehyde.
5. The process of claim 1 wherein the oxidizing agent is chromic acid.
6. The process of claim 1 wherein the acid is sulfuric acid.
7. A compound of the formula

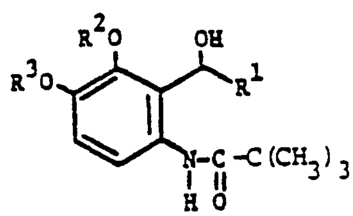

wherein $R_1$, $R_2$ and $R_3$ are lower alkyl.

8. The compound of claim 7 which compound is 2'-(1-hydroxyethyl)-3',4'-dimethoxy-2,2-dimethyl-propioanilide.

## Patentansprüche

1. Verfahren zur Herstellung einer Verbindung der Formel :

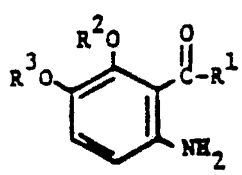

welches das Umsetzen eines substituierten Anilins der Formel :

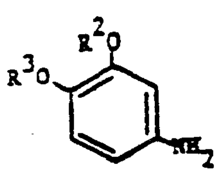

mit einer Pivalinsäureverbindung der Formel :

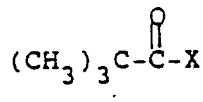

unter Bildung eines Anilids der Formel

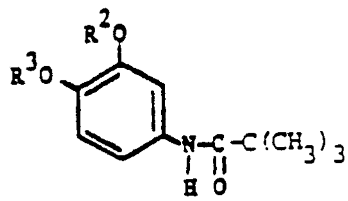

das Umsetzen des Anilids zuerst mit einer Organolithiumverbindung der Formel

R—Li

und das Umsetzen des entstandenen Produktes mit einem Aldehyd der Formel

$R_1$—CHO

unter Bildung eines Anilids der Formel :

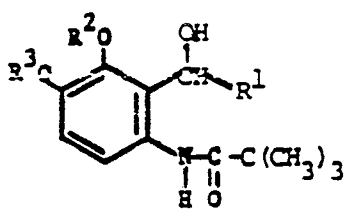

das Umsetzen des Anilids mit einem Oxidationsmittel unter Bildung eines Anilids der Formel :

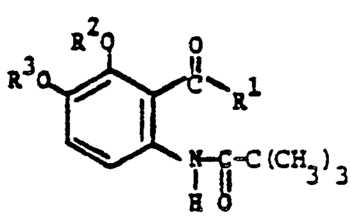

und das Hydrolysieren des Anilids mit einer Säure umfaßt, wobei $R_1$, $R_2$ und $R_3$ Niedrigalkyl sind, $R_2$ und $R_3$ zusammengenommen Methylendioxy sind, und R n-Butyl, sec.-Butyl oder tert.-Butyl ist, und X Chlor, Brom, Fluor oder $OCOC(CH_3)_3$ ist.

2. Verfahren nach Anspruch 1, worin das Säurehalogenid Pivaloylchlorid ist.

3. Verfahren nach Anspruch 1, worin die Organolithiumverbihdung n-Butyllithium ist.

4. Verfahren nach Anspruch 1, worin der Aldehyd Acetaldehyd ist.

5. Verfahren nach Anspruch 1, worin das Oxidationsmittel Chromsäure ist.

6. Verfahren nach Anspruch 1, worin die Säure Schwefelsäure ist.

7. Eine Verbindung der Formel :

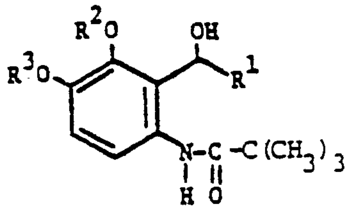

worin $R_1$, $R_2$ und $R_3$ Niedrigalkyl sind.

8. Verbindung nach Anspruch 7, welche Verbindung 2'-(1-Hydroxyethyl)-3',4'-dimethoxy-2,2-dimethylpropioanilid ist.

**Revendications**

1. Procédé pour préparer un composé de formule

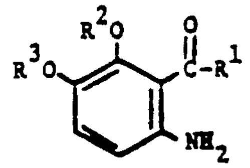

qui comprend la réaction d'une aniline substituée de formule

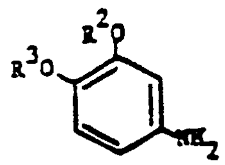

avec un dérivé d'acide pivalique de formule

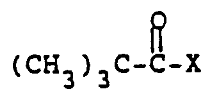

pour former un anilide de formule

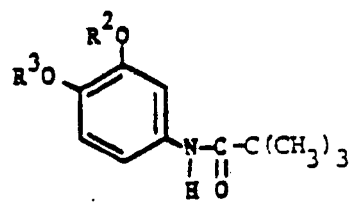

la réaction de l'anilide d'abord avec un composé organolithien de formule

R—Li

et la réaction du produit formé avec un aldéhyde de formule

$R_1$—CHO

pour former un anilide de formule

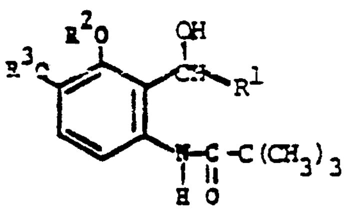

la réaction de l'anilide avec un agent oxydant pour former un anilide de formule

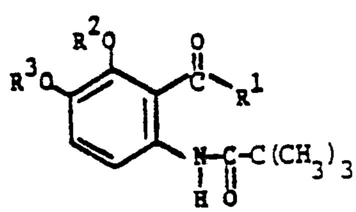

et l'hydrolyse de l'anilide avec un acide, où $R_1$, $R_2$ et $R_3$ sont un alkyle inférieur, $R_2$ et $R_3$, lorsqu'ils sont pris ensemble, sont un méthylènedioxy et R est un n-butyle, un sec-butyle ou un tert-butyle et X est un chloro, bromo, fluoro ou $OCOC(CH_3)_3$.

2. Procédé selon la revendication 1, dans lequel l'halogénure d'acide est le chlorure de pivaloyle.

3. Procédé selon la revendication 1, dans lequel le composé organolithien est le n-butyllithium.

4. Procédé selon la revendication 1, dans lequel l'aldéhyde est l'acétaldéhyde.

5. Procédé selon la revendication 1, dans lequel l'agent oxydant est l'acide chromique.

6. Procédé selon la revendication 1, dans lequel l'acide est l'acide sulfurique.

7. Composé de formule

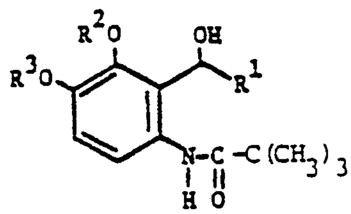

dans laquelle $R_1$, $R_2$ et $R_3$ sont un alkyle inférieur.

8. Composé selon la revendication 7, qui est le 2'-(1-hydroxyéthyl)-3',4'-diméthoxy-2,2-diméthylpropioanilide.